# EUROPÄISCHE PATENTSCHRIFT

(11) **EP 0 439 745 B1**
(45) Veröffentlichungstag und Bekanntmachung des Hinweises auf die Patenterteilung: **03.08.1994**
(21) Anmeldenummer: 90123881.6
(22) Anmeldetag: 12.12.1990
(51) Int. Cl.: C07D 213/61

(54) **Verfahren zur Herstellung von 2-Chlor-5-methyl-pyridin**
Process for the preparation of 2-chloro-5-methyl-pyridine
Procédé de préparation de chloro-2-méthyl-5-pyridine

(30) Priorität: 31.01.1990 DE 4002729; 23.06.1990 DE 4020053
(43) Veröffentlichungstag der Anmeldung: 07.08.1991
(73) Patentinhaber: BAYER AG, 51368 Leverkusen (DE)
(72) Erfinder: Jelich, Klaus, Dr., W-5600 Wuppertal 1 (DE); Kaufmann, Dieter, Dr., W-5060 Bergisch Gladbach 2 (DE); Gallenkamp, Bernd, Dr., W-5600 Wuppertal 1 (DE); Lantzsch, Reinhard, Dr., W-5600 Wuppertal 1 (DE)

(56) Entgegenhaltungen:
- EP-A- 0 324 174
- EP-A- 0 438 691
- FR-A- 1 360 438
- CHEMISTRY OF HETEROCYCLIC COMPOUNDS, Band 14, Supplement, Teil 2, Seiten 111-114, Wiley, New York, US; R.A. ABRAMOVITCH et al.: "Pyridine and itsderivatives"

## Beschreibung

Die Erfindung betrifft ein neues Verfahren zur Herstellung von 2-Chlor-5-methyl-pyridin.

Es ist bekannt, daß man 2-Chlor-5-methyl-pyridin neben 2-Chlor-3-methyl-pyridin, 4-Chlor-3-methyl-pyridin und 3-Chlor-5-methyl-pyridin erhält, wenn man 3-Methyl-pyridin-1-oxid mit Phosphorylchlorid umsetzt (vgl. Weißberger, Chemistry of Heterocyclic Compounds, Pyridine and its Derivatives, Vol. 14, Supplement, Part 2, S. 112). Hauptprodukt dieser Umsetzung ist 4-Chlor-3-methyl-pyridin; der Anteil an 2-Chlor-5-methyl-pyridin liegt im allgemeinen unter 25 %.

Weiter ist bekannt, daß man 2-Chlor-5-methyl-pyridin erhält, wenn man 3-Methyl-pyridin-1-oxid mit Phosphorylchlorid in Gegenwart einer basischen organischen Stickstoffverbindung und in Gegenwart eines Verdünnungsmittels umsetzt (vgl. EP-A 324 174). Die Verwendung von Phosphorylchlorid bedingt den Anfall phosphorhaltiger Abfälle, deren Entsorgung problematisch ist.

Es wurde nun ein neues Verfahren zur Herstellung von 2-Chlor-5-methyl-pyridin der Formel (I) 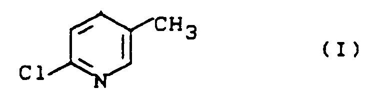
aus 3-Methyl-pyridin-1-oxid der Formel (II) 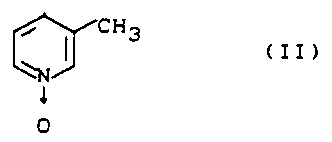
gefunden, welches dadurch gekennzeichnet ist, daß man die Umsetzung in Gegenwart eines Chlorierungsmittels der allgemeinen Formel (III) 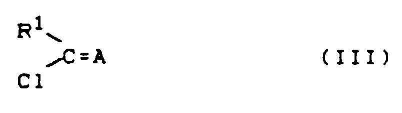
in welcher
- A: für Sauerstoff oder die Gruppierung 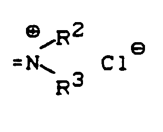 steht, worin
R² und R³ einzeln für C₁-C₈-Alkyl, C₃-C₆-Cycloalkyl oder Phenyl stehen oder zusammen für C₂-C₆-Alkandiyl oder C₂-C₅-Oxaalkandiyl stehen und
- R¹: für Wasserstoff, Chlor, C₁-C₈-Alkyl, C₃-C₆-Cycloalkyl, Phenyl oder die Gruppierung 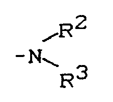 steht, worin R²
und R³ die oben Bedeutungen haben,
und wobei, falls R¹ für Phenyl steht, A zwingend für die Gruppierung 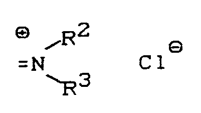 steht,
in Gegenwart einer basischen organischen Stickstoffverbindung und in Gegenwart eines Verdünnungsmittels bei Temperaturen zwischen -20°C und +120°C durchführt und das Reaktionsprodukt in üblicher Weise aufarbeitet, wobei die Umsetzung mit Phosgen in Anwesenheit aromatischer Carbonsäurechlaride ausgenommen ist.

Es ist als überraschend zu bezeichnen, daß 2-Chlor-5-methyl-pyridin nach dem erfindungsgemäßen Verfahren in guten Ausbeuten erhalten werden kann, da die Herstellung dieser und ähnlicher Verbindungen unter Verwendung von Chlorierungsmitteln der Formel (III) nicht bekannt ist und auch durch bisher bekannt gewordene Verfahren nicht nachgelegt wird. Aus EP-OS 438 691 ist die Umsetzung von Pyridin-N-oxiden zu 2-Chlorpyridinen mit Phosgen in Gegenwart eines aromatischen Carbonsäurechlorids bekannt. EP-OS 438 691 stellt Stand der Technik gemäß Artikel 54(3)dar.

Der Vorteil des erfindungsgemäßen Verfahrens gegenüber dem bekannten Stand der Technik liegt insbesondere darin, daß die Chlorierungsmittel der Formel (III) und ihre Umwandlungsprodukte ohne größere Probleme entsorgt werden können.

Das erfindungsgemäße Verfahren stellt somit eine wertvolle Bereicherung des Standes der Technik dar.

Der Reaktionsablauf beim erfindungsgemäßen Verfahren kann beispielsweise durch das nachstehende Formelschema skizziert werden: 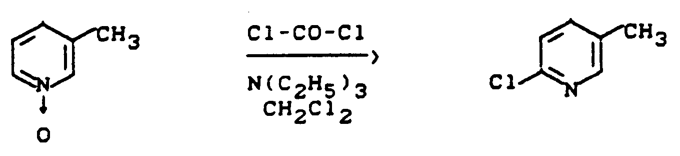
Die Ausgangsverbindung der Formel (II) ist bereits bekannt (vgl. J. Am. Chem. Soc. 76 (1954), 1286-1291).

Die beim erfindungsgemäßen Verfahren zu verwendenden Chlorierungsmittel sind durch die Formel (III) allgemein definiert. In der Formel (III) stehen vorzugsweise
- A: für Sauerstoff oder die Gruppierung 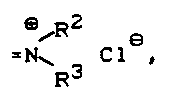 worin R² und R³ einzeln für Methyl, Ethyl, Propyl, Isopropyl, Butyl, Isobutyl, Cyclopentyl, Cyclohexyl oder Phenyl stehen oder zusammen für Butan-1,4-diyl (Tetramethylen), Pentan-1,5-diyl (Pentamethylen) oder 3-Oxa-pentan-1,5-diyl (-CH₂CH₂-O-CH₂CH₂-) stehen und
- R¹: für Wasserstoff, Chlor, Methyl, Ethyl, Propyl, Isopropyl, Butyl, Isobutyl, tert.-Butyl, 1,1,2-Trimethylpropyl, 1,1,3,3-Tetramethylbutyl, Cyclopentyl, Cyclohexyl,
Phenyl oder die Gruppierung 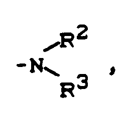 worin R² und R³ die oben als besonders bevorzugt angegebenen Bedeutungen haben,
und wobei, falls R¹ für Phenyl steht, A zwingend für die Gruppierung 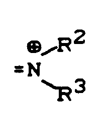
steht.

Ganz besonders bevorzugte Chlorierungsmittel der Formel (III) sind diejenigen, bei denen entweder A für Sauerstoff und R¹ für Chlor steht oder A für die Gruppierung 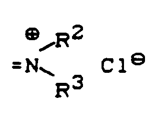
steht, worin R² und R³ für Methyl oder Ethyl stehen, und R¹ für Chlor oder die Gruppierung 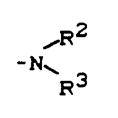
steht, worin R² und R³ für Methyl oder Ethyl stehen.

Als Beispiele für die Chlorierungsmittel der Formel (III) seien genannt:
Phosgen, Dichlormethylen-dimethylimmoniumchlorid und Tetramethyl-chlorformamidiniumchlorid, N,N-Dimethyl-N',N'-diethyl-chlorformamidiniumchlorid und Tetraethylchlorformamidiniumchlorid, Pivaloylchlorid, 2,2,3-Trimethylbuttersäurechlorid und 2,2,4,4-Tetramethylvaleriansäurechlorid.

Die Verbindungen der Formel (III) sind bekannt und/oder können nach an sich bekannten Verfahren hergestellt werden (vgl. Angew. Chem. 72 (1960), 836-845; loc. cit. 81 (1969), 468; loc. cit. 85 (1973), 837-849; Helv. Chim. Acta 42 (1959) 1653-1671).

Das erfindungsgemäße Verfahren wird in Gegenwart einer basischen Stickstoffverbindung durchgeführt. Bevorzugte basische organische Stickstoffverbindungen sind Dialkylamine, wie z.B. Diethylamin, Dipropylamin, Diisopropylamin, Dibutylamin, Diisobutylamin und Di-sec-butylamin, Trialkylamine, wie z.B. Trimethylamin, Triethylamin, Tripropylamin und Tributylamin, Methyldiisopropylamin, Ethyldiisopropylamin, Dialkyl-cycloalkylamine, wie z.B. Dimethyl-cyclopentylamin, Diethyl-cyclopentylamin, Dimethyl-cyclohexylamin und Diethyl-cyclohexylamin, Dialkyl-aralkylamine, wie z.B. Dimethyl-benzylamin und Diethyl-benzylamin sowie Dialkylarylamine, wie z.B. Dimethylanilin.

Besonders bevorzugte basische organische Stickstoffverbindungen sind Trialkylamine, wie z.B. Trimethylamin, Triethylamin, Tripropylamin, Tributylamin, Methyldiisopropylamin und Ethyldiisopropylamin.

Das erfindungsgemäße Verfahren wird in Gegenwart eines Verdünnungsmittels durchgeführt. Als Verdünnungsmittel kommen praktisch alle inerten organischen Lösungsmittel in Frage. Hierzu gehören vorzugsweise gegebenenfalls halogenierte Kohlenwasserstoffe, wie Pentan, Hexan, Heptan, Octan, Cyclohexan, Methylcyclohexan, Petrolether, Benzin, Ligroin, Benzol, Toluol, Xylol, Methylenchlorid, Chloroform, Tetrachlormethan, 1,2-Dichlorethan, 1,1,2-Trichlorethan, 1,2-Dichlorpropan, 1,2,3-Trichlorpropan, Chlorbenzol und Dichlorbenzol, Ether, wie Diethylether, Dipropylether, Diisopropylether, Dibutylether, Methyl-tert-butylether, Methyl-tert.-amylether, Glycoldimethylether, Diglycoldimethylether, Tetrahydrofuran und Dioxan, Ketone, wie Aceton, Methylethylketon, Diethylketon, Methyl-isopropylketon und Methyl-isobutylketon, Ester, wie Essigsäuremethylester, Essigsäureethylester, Essigsäurepropylester, Essigsäurebutylester und Essigsäureamylester, Nitrile, wie Acetonitril und Propionitril, Amide wie Dimethylformamid, Dimethylacetamid und N-Methyl-pyrrolidon, sowie Dimethylsulfoxid und Sulfolan.

Die Reaktionstemperaturen können bei dem erfindungsgemäßen Verfahren in einem größeren Bereich variiert werden. Im allgemeinen arbeitet man bei Temperaturen zwischen -20 °C und +120 °C, vorzugsweise bei Temperaturen zwischen -10 °C und +60 °C.

Das erfindungsgemäße Verfahren wird im allgemeinen unter Normaldruck durchgeführt. Es ist jedoch auch möglich, unter erhöhtem oder vermindertem Druck zwischen 0,1 und 10 bar zu arbeiten.

Zur Durchführung des erfindungsgemäßen Verfahrens setzt man auf 1 Mol 3-Methyl-pyridin-1-oxid der Formel (II) im allgemeinen zwischen 1 und 10 Mol, vorzugsweise zwischen 1,5 und 4,0 Mol, Chlorierungsmittel der Formel (III) und ebenfalls zwischen 1 und 10 Mol, vorzugsweise zwischen 1,5 und 4,0 Mol, der basischen organischen Stickstoffverbindung ein.

In einer bevorzugten Ausführungsform des erfindungsgemäßen Verfahrens werden das 3-Methyl-pyridin-1-oxid und die basische organische Stickstoffverbindung in einem Verdünnungsmittel vorgelegt und das Chlorierungsmittel der Formel (III) wird dann unter Rühren langsam eindosiert. Das komplette Reaktionsgemisch wird dann gegebenenfalls noch weiter bis zum Ende der Umsetzung gerührt.

Die Aufarbeitung kann auf übliche Weise durchgeführt werden. Beispielsweise wird das Reaktionsgemisch mit Wasser verdünnt, gegebenenfalls mit Natronlauge schwach alkalisch gestellt und die organische Phase abgetrennt. Aus der wäßrigen Phase wird mit einem organischen Lösungsmittel, wie z.B. Methylenchlorid, weiteres Produkt extrahiert. Die vereinigten organischen Phasen werden getrocknet und filtriert; vom Filtrat wird das Lösungsmittel im Wasserstrahlvakuum sorgfältig abdestilliert.

Der verbleibende Rückstand enthält im wesentlichen das Produkt der Formel (I), welches auf übliche Weise, beispielsweise durch Vakuumdestillation, weiter gereinigt werden kann.

Das nach dem erfindungsgemäßen Verfahren herstellbare 2-Chlor-5-methyl-pyridin ist als Zwischenprodukt für Pharmazeutika bekannt (vgl. DE-A 2 812 585), kann aber auch als Zwischenprodukt für Insektizide eingesetzt werden (vgl. DE-A 2 630 046).

### Herstellungsbeispiele:

### Beispiel 1

Zu 3,0 g (27,5 mmol) 3-Methyl-pyridin-1-oxid in 50 ml Dichlormethan gibt man 5,6 9 (55,3 mmol) Triethylamin und gibt anschließend unter Eisbadkühlung portionsweise 9,0 g (55,7 mmol) Dichlormethylen-dimethylimmoniumchlorid zu, wobei sich das Gemisch auf 30°C erwärmt. Man läßt für eine Stunde bei Raumtemperatur nachrühren, gibt ca. 50 ml Wasser zu, stellt mit konzentrierter Natronlauge schwach alkalisch und trennt die organische Phase ab. Die wäßrige Phase wird mit Dichlormethan extrahiert. Die vereinigten organischen Phasen werden getrocknet, das Lösungsmittel abgezogen und der Rückstand durch Gaschromatographie analysiert.

Man erhält 3,2 g einer Flüssigkeit, die folgende Zusammensetzung aufweist:
80% 2-Chlor-5-methyl-pyridin (73% d.Th.)
20% 2-Chlor-3-methyl-pyridin (18% d.Th.).

### Beispiel 2

Zu 1,5 g (13,8 mmol) 3-Methyl-pyridin-1-oxid in 30 ml Dichlormethan gibt man 3,6 g (27,8 mmol) N-Ethyl-diisopropylamin und gibt anschließend portionsweise, ohne Kühlung 4,7 g (27,7 mmol) Tetramethyl-chlorformamidiniumchlorid zu, wobei sich das Gemisch auf 32°C erwärmt. Man läßt für 20 Std. nachrühren, gießt den Ansatz in Wasser, trennt die Phase und extrahiert die wäßrige Phase noch einmal mit Dichlormethan.

Die vereinigten organischen Phasen werden getrocknet, das Lösungsmittel abgezogen und der flüssige Rückstand (2,7 g) durch Gaschromatographie analysiert:
43% Tetramethylharnstoff
10% 2-Chlor-3-methyl-pyridin
44% 2-Chlor-5-methyl-pyridin (68% d.Th.)

### Beispiel 3

Zu 5,0 g (45,9 mmol) 3-Methyl-pyridin-1-oxid in 80 ml Dichlormethan gibt man 11,6 g (0,115 mol) Triethylamin und leitet bei Raumtemperatur 11,4 g (0,115 mol) Phosgen ein.

Nach Beendigung des Einleitens wird noch für 1 Stunde bei Raumtemperatur nachgerührt, anschließend ca. 70 ml Wasser zugegeben und mit konzentrierter Natronlauge alkalisch gestellt.

Die organische Phase wird abgetrennt, die wäßrige Phase einmal mit Dichlormethan nachextrahiert und die vereinigten, organischen Phasen getrocknet und eingeengt.

Man erhält 4,9 g eines flüssigen Rückstandes, der folgende gaschromatographisch bestimmte Zusammensetzung aufweist:
64% 2-Chlor-5-methyl-pyridin (54% der Theorie)
11% 2-Chlor-3-methyl-pyridin.

### Herstellungsbeispiel 4

### 2-Chlor-5-methyl-pyridin

Zu einer Lösung von 21,8 g (0,2 Mol) 3-Methylpyridin-1-oxid und 40,4 g (0,4 Mol) Triethylamin in 200 ml Methylenchlorid werden unter Stickstoff 48,2 g (0,4 Mol) Trimethylacetylchlorid in 20 min. getropft. Anschließend wird noch 12 Stunden unter Rückfluß erhitzt, dann abgesaugt, der Filterkuchen mit 50 ml Methylenchlorid gewaschen und das Lösungsmittel abdestilliert. Der Sumpf wird einer Wasserdampfdestillation bei einem pH-Wert von 6 unterworfen. Das Destillat wird dreimal mit je 100 ml Methylenchlorid extrahiert, die Extrakte werden fraktioniert destilliert.
- Ausbeute:: 15,3 g (60 %) eines Gemisches aus 90 % 2-Chlor-5-methylpyridin und 10 % 2-Chlor-3-methylpyridin.

Das reine 2-Chlor-5-methyl-pyridin kann durch fraktionierte Destillation abgetrennt werden.

### Herstellungsbeispiel 5

### 2-Chlor-5-methyl-pyridin

Zu einer Lösung von 21,8 g (0,2 Mol) 3-Methylpyridin-1-oxid und 40,4 g (0,4 Mol) Triethylamin in 200 ml Methylenchlorid werden unter Stickstoff 59,4 g (0,4 Mol) 2,2,3-Trimethylbuttersäurechlorid in 20 min. getropft. Anschließend wird noch 12 Stunden unter Rückfluß erhitzt, dann abgesaugt, der Filterkuchen mit 50 ml Methylenchlorid gewaschen und das Lösungsmittel abdestilliert. Der Sumpf wird einer Wasserdampfdestillation bei einem pH-Wert von 6 unterworfen. Das Destillat wird dreimal mit je 100 ml Methylenchlorid extrahiert, die Extrakte werden fraktioniert destilliert.
- Ausbeute:: 17,9 g (70 %) eines Gemisches aus 93 % 2-Chlor-5-methylpyridin und 7 % 2-Chlor-3-methylpyridin.

Das reine 2-Chlor-5-methyl-pyridin kann durch fraktionierte Destillation abgetrennt werden.

### Herstellungsbeispiel 6

### 2-Chlor-5-methyl-pyridin

Zu einer Lösung von 21,8 g (0,2 Mol) 3-Methylpyridin-1-oxid und 40,4 g (0,4 Mol) Triethylamin in 200 ml Methylenchlorid werden unter Stickstoff 70,4 g (0,4 Mol) 2,2,4,4-Tetramethylvaleriansäurechlorid in 20 min. getropft. Anschließend wird noch 12 Stunden unter Rückfluß erhitzt, dann abgesaugt, der Filterkuchen mit 50 ml Methylenchlorid gewaschen und das Lösungsmittel abdestilliert Der Sumpf wird einer Wasserdampfdestillation bei einem pH-Wert von 6 unterworfen. Das Destillat wird dreimal mit je 100 ml Methylenchlorid extrahiert, die Extrakte werden fraktioniert destilliert.
- Ausbeute:: 17,9 g (70 %) eines Gemisches aus 95 % 2-Chlor-5-methylpyridin und 5 % 2-Chlor-3-methylpyridin.

Das reine 2-Chlor-5-methyl-pyridin kann durch fraktionierte Destillation abgetrennt werden.

## Patentansprüche

1. Verfahren zur Herstellung von 2-Chlor-5-methyl-pyridin der Formel (I) 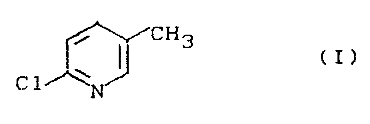 aus 3-Methyl-pyridin-1-oxid der Formel (II) 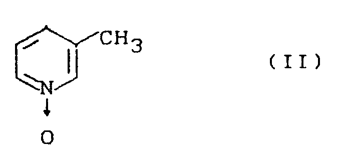 und einem Chlorierungsmittel, dadurch gekennzeichnet, daß man die Umsetzung in Gegenwart eines Chlorierungsmittels der allgemeinen Formel 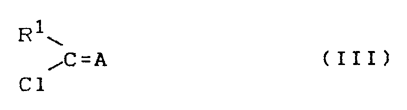 in welcher
A für Sauerstoff oder die Gruppierung 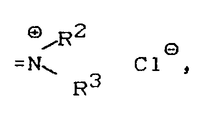 steht,
worin R² und R³ einzeln für C₁-C₈-Alkyl, C₃-C₆-Cycloalkyl oder Phenyl stehen oder zusammen für C₂-C₆-Alkandiyl oder C₂-C₅-Oxaalkandiyl stehen und
R¹ für Wasserstoff, Chlor, C₁-C₈-Alkyl, C₃-C₆-Cycloalkyl, Phenyl oder die Gruppierung 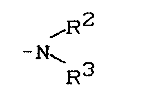 steht, worin R²
und R³ die oben Bedeutungen haben,
und wobei, falls R¹ für Phenyl steht, A zwingend für die Gruppierung 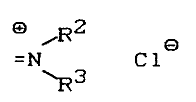 steht,
in Gegenwart einer basischen organischen Stickstoffverbindung und in Gegenwart eines Verdünnungsmittels bei Temperaturen zwischen -20°C und +120°C durchführt und das Reaktionsprodukt in üblicher Weise aufarbeitet, wobei die Umsetzung mit Phosgen in Anwesenheit von aromatischen Carbonsäurechloriden ausgenommen ist.

2. Verfahren gemäß Anspruch 1, dadurch gekennzeichnet, daß man mit einem Chlorierungsmittel der Formel (III) gemäß Anspruch 1 arbeitet,
in welcher
A für Sauerstoff oder die Gruppierung 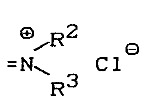 steht,
worin R² und R³ einzeln für Methyl, Ethyl, Propyl, Isopropyl, Butyl, Isobutyl, Cyclopentyl, Cyclohexyl oder Phenyl stehen oder zusammen für Butan-1,4-diyl (Tetramethylen), Pentan-1,5-diyl (Pentamethylen) oder 3-Oxapentan-1,5-diyl (-CH₂CH₂-O-CH₂CH₂-) stehen und
R¹ für Wasserstoff, Chlor, Methyl, Ethyl, Propyl, Isopropyl, Butyl, tert.-Butyl, 1,1,2-Trimethylpropyl, 1,1,3,3-Tetramethylbutyl, Isobutyl, Cyclopentyl, Cyclohexyl,
Phenyl oder die Gruppierung 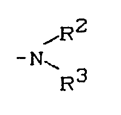 steht,
worin R² und R³ die oben angegebenen Bedeutungen haben, und wobei, falls R¹ für Phenyl steht,
A zwingend für die Gruppierung 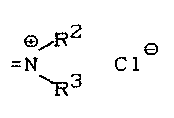 steht.

3. Verfahren gemäß Anspruch 1, dadurch gekennzeichnet, daß man mit einem Chlorierungsmittel der Formel (III) gemäß Anspruch 1 arbeitet,
in welcher
entweder A für Sauerstoff und R¹ für Chlor steht oder A für die Gruppierung 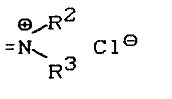 steht, worin R² und R³ für Methyl oder Ethyl stehen, und R¹ für Chlor oder die Gruppierung 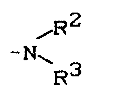 steht, worin R² und R³ für Methyl oder Ethyl stehen.

4. Verfahren gemäß Anspruch 1, dadurch gekennzeichnet, daß man mit einem Chlorierungsmittel der folgenden Reihe arbeitet: Phosgen, Dichlormethylen-dimethylimmoniumchlorid und Tetramethyl-chlorformamidiniumchlorid, N,N-Dimethyl-N',N'-diethyl-chlorformamidiniumchlorid und Tetraethylchlorformamidiniumchlorid, Pivaloylchlorid, 2,2,3-Trimethylbuttersäurechlorid, 2,2,4,4-Tetramethylvaleriansäurechlorid.

5. Verfahren gemäß Anspruch 1 bis 4, dadurch gekennzeichnet, daß man in Gegenwart einer basischen organischen Stickstoffverbindung der folgenden Reihe arbeitet; Dialkylamine, Trialkylamine, Dialkyl-cycloalkylamine, Dialkyl-aralkylamine, sowie Dialkylarylamine.

6. Verfahren gemäß Anspruch 1 bis 5, dadurch gekennzeichnet, daß man in Gegenwart einer basischen organischen Stickstoffverbindung der folgenden Reihe arbeitet; Diethylamin, Dipropylamin, Diisopropylamin, Dibutylamin, Diisobutylamin, Di-sec-butylamin, Trimethylamin, Triethylamin, Tripropylamin, Tributylamin, Methyldiisopropylamin, Ethyldiisopropylamin, Dimethyl-cyclopentylamin, Diethyl-cyclopentylamin, Dimethyl-cyclohexylamin, Dimethylbenzylamin, Diethylbenzylamin, Dimethylanilin.

7. Verfahren gemäß Anspruch 1 bis 6, dadurch gekennzeichnet, daß man in Gegenwart eines inerten organischen Lösungsmittels als Verdünnungsmittel arbeitet.

8. Verfahren gemäß Anspruch 1 bis 7, dadurch gekennzeichnet, daß man bei einer Temperatur zwischen -10°C und +60°C arbeitet.

9. Verfahren gemäß Anspruch 1 bis 8, dadurch gekennzeichnet, daß man auf 1 Mol 3-Methyl-pyridin-1-oxid der Formel (II) zwischen 1 und 10 Mol Chlorierungsmittel der Formel (III) und zwischen 1 und 10 Mol der basischen organischen Stickstoffverbindung einsetzt.

10. Verfahren gemäß Anspruch 1 bis 9, dadurch gekennzeichnet, daß man das 3-Methyl-pyridin-1-oxid der Formel (II) und die basische Stickstoffverbindung in einem Verdünnungsmittel vorlegt und das Chlorierungsmittel der Formel (III) unter Rühren langsam eindosiert und das komplette Reaktionsgemisch gegebenfalls noch weiter bis zum Ende der Umsetzung rührt.

## Claims

1. Process for the preparation of 2-chloro-5-methylpyridine of the formula (I) 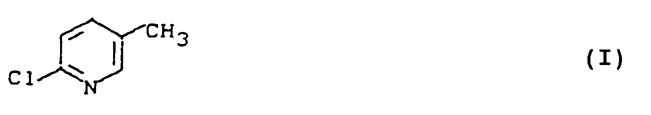 from 3-methyl-pyridine-1-oxide of the formula (II) 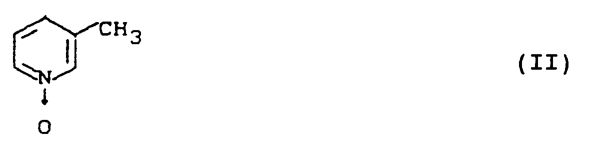 and a chlorinating agent, characterized in that the reaction is carried out in the presence of a chlorinating agent of the general formula 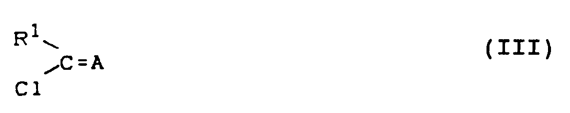 in which
A represents oxygen or the grouping 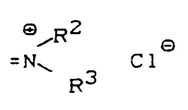
in which R² and R³ individually represent C₁-C₈-alkyl, C₃-C₆-cycloalkyl or phenyl or together represent C₂-C₆-alkanediyl or C₂-C₅-oxaalkanediyl and
R¹ represents hydrogen, chlorine, C₁-C₈-alkyl, C₃-C₆-cycloalkyl, phenyl or the grouping 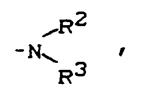 in which R² and R³ have the abovementioned meanings
and where, if R¹ represents phenyl, A compulsorily represents the grouping 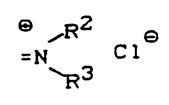
in the presence of a basic organic nitrogen compound and in the presence of a diluent at temperatures between -20°C and +120°C, and the reaction product is worked up in the customary manner, with the exception of the reaction with phosgene in the presence of aromatic carbonyl chlorides.

2. Process according to Claim 1, characterized in that the reaction is carried out using a chlorinating agent of the formula (III) according to Claim 1,
in which
A represents oxygen or the grouping 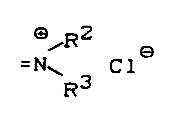 in which R² and R³ individually represent methyl, ethyl, propyl, isopropyl, butyl, isobutyl, cyclopentyl, cyclohexyl or phenyl or together represent butane-1,4-diyl (tetramethylene), pentane-1,5-diyl (pentamethylene) or 3-oxapentane-1,5-diyl (-CH₂CH₂-O-CH₂CH₂-) and
R¹ represents hydrogen, chlorine, methyl, ethyl, propyl, isopropyl, butyl, tert.-butyl, 1,1,2-trimethylpropyl, 1,1,3,3-tetramethylbutyl, isobutyl, cyclopentyl, cyclohexyl, phenyl or
the grouping 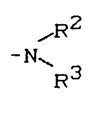
in which R² and R³ have the abovementioned meanings and where, if R¹ represents phenyl,
A compulsorily represents the grouping

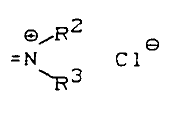

3. Process according to Claim 1, characterized in that the reaction is carried out using a chlorinating agent of the formula (III) according to Claim 1,
in which
A either represents oxygen and R¹ represents chlorine or A represents the grouping 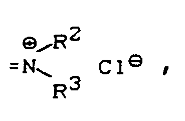 in which R² and R³ represent methyl or ethyl, and R¹ represents chlorine or the grouping 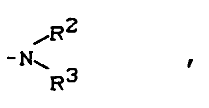 in which R² and R³ represent methyl or ethyl.

4. Process according to Claim 1, characterized in that the reaction is carried out using a chlorinating agent from the following series: phosgene, dichloromethylene-dimethylimmonium chloride and tetramethylchloroformamidiniumchloride, N,N-dimethyl-N',N'-diethyl-chloroformamidinium chloride and tetraethyl-chloroformamidinium chloride, pivaloyl chloride, 2,2,3-trimethylbutyryl chloride and 2,2,4,4-tetramethylvaleryl chloride.

5. Process according to Claim 1 to 4, characterized in that the reaction is carried out in the presence of a basic organic nitrogen compound from the following series: dialkylamines, trialkylamines, dialkylcycloalkylamines, dialkyl-aralkylamines and dialkylarylamines.

6. Process according to Claim 1 to 5, characterized in that the reaction is carried out in the presence of a basic organic nitrogen compound from the following series: diethylamine, dipropylamine, diisopropylamine, dibutylamine, diisobutylamine, di-sec.-butylamine, trimethylamine, triethylamine, tripropylamine, tributylamine, methyldiisopropylamine, ethyldiisopropylamine, dimethyl-cyclopentylamine, diethyl-cyclopentylamine, dimethyl-cyclohexylamine, dimethyl-benzylamine, diethyl-benzylamine and dimethylaniline.

7. Process according to Claim 1 to 6, characterized in that the reaction is carried out in the presence of an inert organic solvent as diluent.

8. Process according to Claim 1 to 7, characterized in that the reaction is carried out at a temperature between -10°C and +60°C.

9. Process according to Claim 1 to 8, characterized in that between 1 and 10 moles of chlorinating agent of the formula (III) and between 1 and 10 moles of the basic organic nitrogen compound are employed per mole of 3-methyl-pyridine-1-oxide of the formula (II).

10. Process according to Claim 1 to 9, characterized in that the 3-methyl-pyridine-1-oxide of the formula (II) and the basic nitrogen compound are initially introduced in a diluent and the chlorinating agent of the formula (III) is slowly metered in with stirring and the complete reaction mixture is optionally further stirred until the reaction is complete.

## Revendications

1. Procédé de préparation de la 2-chloro-5-méthyl-pyridine de formule I 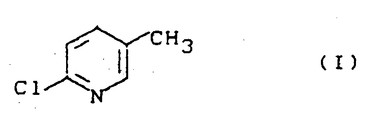 à partir du 1-oxyde de 3-méthyl-pyridine de formule II 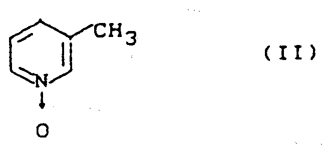 et d'un agent chlorant, caractérisé en ce que l'on effectue la réaction en présence d'un agent chlorant de formule générale 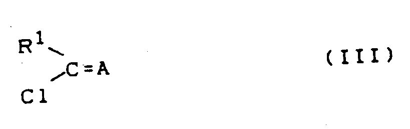 dans laquelle
A représente l'oxygène ou le groupement 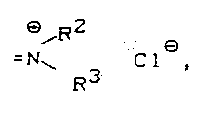 dans lequel R² et R³ représentent chacun un groupe alkyle en C₁-C₈, cycloalkyle en C₃-C₆ ou phényle ou forment ensemble un groupe alcane-diyle en C₂-C₆ ou oxa-alcane-diyle en C₂-C₅ et
R₁ représente l'hydrogène, le chlore, un groupe alkyle en C₁-C₈, cycloalkyle en C₃-C₆, phényle ou le groupement 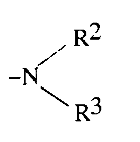 dans lequel R² et R³ ont les significations indiquées ci-dessus,
sous réserve que, lorsque R¹ représente un groupe phényle, A représente obligatoirement le groupement 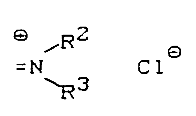 en présence d'un composé organique azoté basique et en présence d'un diluant, à des températures allant de -20 à +120°C, et on isole le produit de réaction de la manière habituelle, la réaction avec le phosgène en présence de chlorures d'acides carboxyliques aromatiques étant exclue.

2. Procédé selon revendication 1, caractérisé en ce que l'on opère avec un agent chlorant de formule III de la revendication 1 dans laquelle
A représente l'oxygène ou le groupement 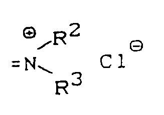 dans laquelle R² et R³ représentent chacun un groupe méthyle, éthyle, propyle, isopropyle, butyle, isobutyl, cyclopentyle, cyclohexyle ou phényle ou forment ensemble un groupe butane-1,4-diyle (tétraméthylène), pentane-1,5-diyle(pentaméthylène) ou 3-oxa-pentane-1,5-diyle (-CH₂CH₂-O-CH₂CH₂-) et
R¹ représente l'hydrogène, le chlore, un groupe méthyle, éthyle, propyle, isopropyle, butyle, tert-butyle, 1,1,2-triméthylpropyle, 1,1,3,3-tétraméthylbutyle, isobutyle, cyclopentyle, cyclohexyle, phényle ou le groupement 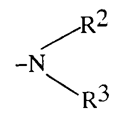 dans lequel R² et R³ ont les significations indiquées ci-dessus, sous réserve que, lorsque R¹ représente un groupe phényle,
A représente obligatoirement le groupement

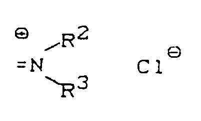

3. Procédé selon revendication 1, caractérisé en ce que l'on opère avec un agent chlorant de formule III de la revendication 1 dans laquelle
A représente l'oxygène et R¹ le chlore, ou bien A représente le groupement 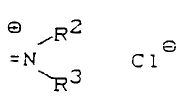 dans lequel R² et R³ représentent des groupes méthyle ou éthyle, et R¹ représente le chlore ou le groupement 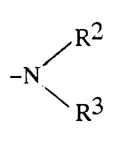 dans lequel R² et R³ représentent des groupes méthyle ou éthyle.

4. Procédé selon la revendication 1, caractérisé en ce que l'on opère avec un agent chlorant choisi parmi les suivants : phosgène, chlorure de dichlorométhylène-diméthylimmonium et chlorure de tétraméthyl-chloroformamidinium, chlorure de N,N-diméthyl-N',N'-diéthylchloroformamidinium et chlorure de tétraéthylchloroformamidinium, chlorure de pivaloyle, chlorure de l'acide 2,2,3-triméthyl-butyrique, chlorure de l'acide 2,2,4,4-tétraméthylvalérique.

5. Procédé selon les revendications 1 à 4, caractérisé en ce que l'on opère en présence d'un composé organique azoté basique choisi parmi les suivants : dialkylamines, trialkylamines, dialkyl-cycloalkylamines, dialkylaralkylamines et dialkyl-arylamines.

6. Procédé selon les revendications 1 à 5, caractérisé en ce que l'on opère en présence d'un composé organique azoté basique choisi parmi les suivants : diéthylamine, dipropylamine, diisopropylamine, dibutylamine, diisobutylamine, di-sec-butylamine, triméthylamine, triéthylamine, tripropylamine, tributylamine, méthyldiisopropylamine, éthyldiisopropylamine, diméthylcyclopentylamine, diéthylcyclopentylamine, diméthlcyclohexylamine, diméthylbenzy-lamine, diéthylbenzylamine, diméthylaniline.

7. Procédé selon les revendications 1 à 6, caractérisé en ce que l'on opère en présence d'un solvant organiques inerte qui sert de diluant.

8. Procédé selon les revendications 1 à 7, caractérisé en ce que l'on opère à une température comprise entre - 10 et +60°C.

9. Procédé selon les revendications 1 à 8, caractérisé en ce que l'on met en oeuvre de 1 à 10 mol de l'agent chlorant de formule III et de 1 à 10 mol du composé organique azoté basique par mole du 1-oxyde de 3-méthyl-pyridine de formule II.

10. Procédé selon les revendications 1 à 9, caractérisé en ce que l'on ajoute lentement, sous agitation, l'agent chlorant de formule III au 1-oxyde de 3-méthyl-pyridine de formule II et au composé azoté basique dans un diluant et le cas échéant on agite encore le mélange de réaction complet jusqu'à la fin de la conversion.
